(19) Europäisches Patentamt

European Patent Office

Office européen des brevets

(11) Numéro de publication: **0 273 780 B1**

(12) **FASCICULE DE BREVET EUROPEEN**

(45) Date de publication de fascicule du brevet: **17.03.93**  (51) Int. Cl.⁵: **C07K 3/24**

(21) Numéro de dépôt: **87401625.6**

(22) Date de dépôt: **09.07.87**

(54) **Procédé d'isolation de protides biologiquement actifs, stables en milieu acide.**

(30) Priorité: **20.11.86 FR 8616166**

(43) Date de publication de la demande:
**06.07.88 Bulletin 88/27**

(45) Mention de la délivrance du brevet:
**17.03.93 Bulletin 93/11**

(84) Etats contractants désignés:
**BE CH DE ES GB IT LI NL SE**

(56) Documents cités:
**WO-A-82/01641**
**DE-B- 1 251 911**
**FR-A- 1 576 763**

**CHEMICAL ABSTRACTS, vol. 93, no. 5, 4 août 1980, page 380, no. 40199y, Columbus, Ohio, US; C. HAMELIN et al.: "Coprecipitation of topoisomerase activity with simian virus 40 nucleoprotein complexes by divalent cations", & BIOCHIMIE 1980, 62(4), 261-5**

(73) Titulaire: **Amiguet, Pierre**
**Ruelle de Cojonnex 8**
**CH-1807 Blonay(CH)**

(72) Inventeur: **Amiguet, Pierre**
**Ruelle de Cojonnex 8**
**CH-1807 Blonay(CH)**

(74) Mandataire: **Beauchamps, Georges et al**
**Cabinet Z.Weinstein 20, avenue de Friedland**
**F-75008 Paris (FR)**

**Description**

La présente invention a pour objet un procédé d'isolation de protides biologiquement actifs, stables en milieu acide, d'origine animale, végétale ou microbienne.

L'invention trouve notamment application pour l'isolation, à l'échelle industrielle, de certains inhibiteurs de protéase polyvalents à faible poids moléculaire, dont on a décelé récemment la possible utilisation dans l'industrie pharmaceutique et alimentaire.

Jusqu'à présent, les difficultés de l'isolement de tels protides nécessitent l'utilisation de différentes techniques de chromatographie qui présentent l'inconvénient non seulement d'être très lentes, et très coûteuses, mais dont l'application à grande échelle est limitée.

On connaît par ailleurs un procédé pour produire un isolat de protéines à partir du petit lait, décrit dans la demande de brevet internationale WO 82/01641, passant par une étape d'alcalinisation et d'acidification. Mais l'ensemble des protéines est isolé sans distinction.

L'objet essentiel de la présente invention est de proposer un procédé d'isolation de protides biologiquement actifs, stables en milieu acide, contenant moins d'environ 10% en poids, à l'état sec, d'agents contaminants non protéiniques, pouvant être mis en oeuvre de façon simple et peu coûteuse et qui rend particulièrement intéressante son exploitation à grande échelle.

Pour atteindre ce but, le procédé d'isolation conforme à la présente invention est caractérisé en ce qu'il comprend :

- la co-précipitation par acidification en solution du protide à isoler et d'au moins une protéine sensible à l'acide, présentant un poids moléculaire supérieur à celui du protide et supérieur à 20 000 et un point isoélectrique compris entre 1 et 5, et étant insoluble à son point isoélectrique ;

l'isolation dudit protide du précipité ainsi formé, par mise en suspension, dans une solution aqueuse, dudit précipité et séparation subséquente des matières insolubles en suspension ;

et éventuellement, l'élimination subséquente des contaminants de faible poids moléculaire.

Ce procédé très simple permet l'isolation et donc la production de protides biologiquement actifs en grandes quantités.

Selon une caractéristique particulière de l'invention, ce procédé consiste, préalablement à l'étape de co-précipitation précitée, à purifier le protide stable à l'acide jusqu'à un degré de pureté d'au moins 1%, et de préférence supérieur à 10%, par toute opération de purification habituelle à l'exception des opérations basées sur des techniques chromatographiques telles que perméation sur gel, échange d'ions, chromatographie par affinité.

L'invention sera mieux comprise, et d'autres buts, caractéristiques, détails et avantages de celle-ci apparaîtront plus clairement à la lumière de la description explicative qui va suivre.

Dans cette description, le terme "protide" désigne soit un peptide, soit une protéine, soit des composés glyco-conjugués de ceux-ci.

Par ailleurs, on désigne par l'expression "protide stable en milieu acide" tout protide dont la structure et l'activité biologique ne sont pas altérées de façon irréversible dans un milieu acide ou sous l'action d'un agent chimique acide.

L'étape essentielle du procédé conforme à l'invention est la co-précipitation, en solution ou en suspension, du protide, et d'au moins une protéine sensible à l'acide. La présente invention est en effet basée sur la découverte que des protides stables en milieu acide sont co-précipités par acidification en présence d'une protéine sensible à l'acide, présentant de préférence un poids moléculaire supérieur à celui du protide, et que l'on peut ainsi isoler ces protides d'une phase liquide par centrifugation ou filtration. Cette opération est impossible en l'absence d'une protéine sensible à l'acide, puisque les protides stables en milieu acide restent alors en solution après acidification.

L'invention est de plus basée sur la découverte de la possibilité, après l'étape de co-précipitation précitée, d'isoler les protides dans un état de pureté très avancé (protides contenant moins d'environ 10% en poids d'agents contaminants non protéiniques), par mise en suspension du précipité de préférence dans une solution aqueuse. Par cette opération, les protides sont libérés en solution et peuvent être ainsi isolés par élimination des matières insolubles encore présentes, notamment par centrifugation ou filtration.

On entend par "co-précipitation" l'opération selon laquelle une substance soluble est précipitée par le seul fait qu'elle se trouve physiquement emprisonnée dans une substance rendue insoluble.

Dans le procédé conforme à la présente invention, cette opération poursuit, comme on le comprendra par la suite, un double but :

- l'élimination des contaminants solubles qui restent dans le surnageant,
- l'élimination de toutes les protéines contaminantes à poids moléculaire supérieur à environ 5000, par leur dénaturation irréversible.

Le procédé conforme à la présente invention vise essentiellement l'isolation de protides présentant une structure rigide. Généralement, cette rigidité est due à la présence, dans la structure du protide d'une ou plusieurs liaisons entre deux atomes de soufre, communément désignées par "pont disulfure". Ces ponts sont réalisés entre deux cystéines présentes dans la molécule en formant ainsi un résidu de cystine (ou dicystéine).

On a découvert, de façon expérimentale, qu'il existait une relation entre la stabilité en milieu acide d'un protide et la teneur en cystine présent dans la molécule. Ainsi, on estime qu'un protide a une stabilité accrue en milieu acide à partir d'une teneur en demi-cystine égale à environ 2% par rapport au nombre total d'acides aminés. Cependant cette condition tout en étant une condition nécessaire n'est pas une condition suffisante. On doit donc déterminer expérimentalement dans chaque cas si un protide particulier est effectivement stable en milieu acide.

A titre d'exemple non limitatif, le tableau 1 donne un certain nombre de protides qui satisfont à l'exigence structurelle précitée.

TABLEAU 1.

| Protide | Nombre total d'acides aminés | ponts disulfure | demi-cystine (%) |
|---|---|---|---|
| Calcitonine | 14 | 1 | 14 |
| Insuline | 55 | 3 | 11 |
| Inhibiteur de trypsine de Kazal | 56 | 3 | 11 |
| Inhibiteur de trypsine de Kunitz, aprotinine | 58 | 3 | 10 |
| Inhibiteur de trypsine colostral | 67 | 3 | 9 |
| Inhibiteur de Bowman-Birk | 71 | 7 | 20 |
| Proinsuline | 81 | 3 | 7 |
| Neurophysine | 97 | 7 | 14 |
| Ribonucléase | 124 | 4 | 6 |
| Lysozyme | 129 | 4 | 6 |
| Inhibiteur de trypsine du soja de Kunitz | 182 | 2 | 2 |

Il est notable que le nombre de protides stables en milieu acide est très limité, si on le compare au nombre de protéines connues.

Il est d'autant plus surprenant de constater que malgré le nombre restraint de protides stables à l'acide, deux sont déjà couramment utilisés comme médicaments, à savoir l'insuline et l'aprotinine, et d'autres actuellement expérimentés pour déterminer leur possible effet dans le traitement de maladies variées.

En outre, certaines sources de protides contiennent plusieurs de ces protides qui sont alors simultanément isolés. Il en est ainsi du colostrum qui contient l'inhibiteur colostral et l'alpha-lactalbumine, un homologue de la lysozyme.

Le tableau 1 montre également que ce sont des inhibiteurs de protéase de bas poids moléculaire, qui constituent la majorité des protides naturels stables à l'acide. D'autres inhibiteurs, constituant pour la plupart des homologues génétiques de ceux donnés au tableau 1, ont été isolés de sources aussi variées que pommes de terre, escargots, oeufs de tortue et venin de serpent. On connait depuis fort longtemps les propriétés exceptionnelles de résistance en milieu acide et à la dénaturation à la chaleur de ce groupe de protéines qui conviennent tout particulièrement pour la mise en oeuvre du procédé d'isolation conforme à l'invention.

Selon une caractéristique de l'invention, la co-précipitation est réalisée en utilisant des extraits bruts ou des solutions contenant un protide stable à l'acide pré-purifiées.

L'opération de pré- purification dépend de l'origine du protide à isoler. Généralement, les huiles et graisses doivent être éliminées en premier lieu si l'on utilise comme source du lait, une graine oléagineuse ou des organes. Cette purification est réalisée en suivant des processus traditionnels, tels que par exemple une centrifugation dans le cas du lait ou une extraction au solvant dans le cas des huiles oléagineuses et des organes d'animaux, en utilisant des solvants tels que l'acétone, l'hexane ou certains hydrocarbures halogénés.

Une autre purification préliminaire peut être réalisée, si on le souhaite, par filtration, centrifugation et autres opérations analogues, ainsi que par combinaison de ces opérations et récupération consécutive de la phase solide ou liquide qui contient la substance à isoler.

Ces opérations de purification préalable peuvent également comprendre des opérations telles que ultra et diafiltration, électrodialyse, précipitation par sulfate d'ammonium et toute autre opération analogue, ainsi que leurs combinaisons. Cependant, comme on le comprend, on évitera toute opération de purification basée sur des techniques chromatographiques, telles que perméation sur gel, échange d'ions, interaction hydrophobe, ou chromatographie par affinité, puisque ces techniques sont lentes, coûteuses et fragiles de sorte qu'elles ne se prêtent guère à une utilisation à l'échelle industrielle.

Les procédés connus d'isolation de protéines biologiquement actives utilisent sans exception une ou plusieurs étapes de chromatographie. La littérature montre qu'il en est de même des procédés d'isolation d'inhibiteurs de protéase à faible poids moléculaire, dont on sait qu'ils présentent des structures particulièrement rigides. L'étape de co-précipitation conforme au procédé de l'invention pour l'isolation de protides stables en milieu acide remplace de façon particulièrement avantageuse les étapes de chromatographie prescrites dans les publications et brevets antérieurs relatifs à l'isolation de protides biologiquement actifs purs. Le procédé d'isolation conforme à la présente invention permet donc des gains de temps et de coût très importants qui rendent particulièrement avantageuse son utilisation à l'échelle industrielle.

L'étape de purification préalable est destinée à amener les protides dans des conditions plus favorables pour l'étape de co-précipitation subséquente. A cet effet, le protide est généralement purifié jusqu'à un degré de pureté d'au moins 1%, et de préférence supérieur à 10% sur la base de son poids sec. De plus, la solution ou suspension pré-purifiée contenant le protide stable à l'acide doit présenter de préférence une teneur totale en matière sèche, y compris la protéine sensible à l'acide, d'au moins 5%, et n'excédant pas environ 30%, de préférence d'environ 15 à 20%.

Conformément à l'invention, la protéine sensible à l'acide peut être soit exogène, comme par exemple une protéine du lait ajoutée à un extrait de pancréas de porc, soit endogène comme par exemple une protéine de soja ajoutée à un extrait de soja.

Des protéines convenant particulièrement à l'étape de co-précipitation seront choisies parmi les protéines de poids moléculaire supérieur à 20000, présentant de préférence un point iso-électrique compris entre 1 et 5, et qui sont insolubles à leur point isoélectrique dans l'eau présentant une force ionique de moins d'environ 0,1.

On choisira bien entendu une protéine disponible en grande quantité et à bas prix.

La protéine sensible à l'acide préféré, est la caséine, et par exemple la caséine disponible dans le commerce sous la désignation "caséine Hammersten" après neutralisation. On peut également utiliser une protéine de réserve de graine oléagineuse telle que glycinine et conglycinine du soja. Ces protéines sont également disponibles dans le commerce sous la désignation "isolat de soja".

Ces protéines préférentielles sont obtenues par dégraissage de lait et soja, respectivement, et précipitation subséquente de la protéine d'une solution aqueuse au pH de leur point isoélectrique (pH 4,5).

Selon une caractéristique particulière de l'invention, la protéine sensible à l'acide précité est ajoutée à une solution ou suspension contenant le protide à isoler sous forme sèche, ou sous forme d'une solution concentrée. La concentration finale devra être de 1 à 15% en poids par volume et de préférence de 5 à 10%.

La co-précipitation est réalisée par acidification rapide de la solution ou de la suspension contenant le protide à isoler, en ajoutant une quantité efficace d'un acide fort jusqu'à l'obtention d'un pH compris entre 0 et 5, et de préférence entre 1,5 et 3,5.

Un acide préféré est un acide susceptible de dénaturer la protéine sensible à l'acide, c'est-à-dire susceptible de rendre, de façon irréversible, cette protéine insoluble. A cet égard, l'acide préférentiel est l'acide trichloroacétique.

Le co-précipité obtenu est récupéré par centrifugation ou filtration, puis mis à nouveau en suspension dans environ 10 volumes d'une solution aqueuse, constituée de préférence par de l'eau distillée. La matière insoluble est alors finement dispersée par brassage vigoureux au moyen d'un mélangeur approprié, et le protide stable à l'acide est alors libéré dans la solution. On peut alors séparer les matières insolubles en suspension par centrifugation ou filtration et concentrer la phase soluble contenant le protide, par exemple par évaporation, en obtenant ainsi un concentrat ne contenant pratiquement pas d'agents contaminants à poids moléculaire élevé supérieur à environ 5000 et moins d'environ 10% en poids de matière dialysable à faible poids moléculaire. Ces matières peuvent être facilement éliminées par exemple par dialyse.

Il est à noter que l'utilisation de l'acide trichloroacétique est particulièrement avantageuse, puisque cet acide a de plus la propriété d'éliminer les germes éventuellement présents dans les protides. On sait en effet que les bactéries et virus ont besoin, pour leur croissance, de protéines constitutives. Ainsi, l'invention permet d'obtenir des protides présentant une teneur en germe très réduite, puisque lors de l'étape de co-précipitation, ces germes sont tués par l'acide trichloroacétique et éliminés de la suspension en même temps que les autres matières insolubles.

Les exemples suivants, donnés uniquement à titre illustratif permettront de mieux apprécier les avantages du procédé conforme à l'invention.

Exemple 1.

Isolation de l'inhibiteur de protéase polyvalent de Bowman-Birk, à partir du soja.

Des graines de soja décortiquées (variété Fiskeby V, Thompson et Morgan (Ipswich) Ltd.) ont été finement broyées et dégraissées par extraction à l'hexane dans un appareil de Shoxlett. 500 g de la poudre ainsi obtenue, qui contenait moins de 1% de graisse, ont été soumis deux fois à extraction, avec 10 volumes d'une solution contenant 7 parties d'éthanol et 3 parties d'eau (vol/vol). Après centrifugation, le surnageant a été éliminé et le résidu a subi deux extractions avec une solution contenant 4 parties d'éthanol et 6 parties d'eau (vol/vol). Après centrifugation, le sédiment a été éliminé. Les surnageants collectés et réunis ont été concentrés environ 10 fois sous vide, la température étant maintenue en dessous de 50°C. Ensuite, la solution jaunâtre a été clarifiée par centrifugation et filtration et a été soumise à ultrafiltration sur une membrane UM 10 de Diaflo. Le perméat contenant environ 90% de matière sèche a été éliminé. Le rétentat d'environ 100 ml a été soumis à une diafiltration avec un volume d'environ 100 ml, et concentré jusqu'à l'obtention d'une concentration en matière sèche d'environ 10%.

On a ajouté à cette solution, de la caséinate de sodium lyophilisée, pour obtenir une concentration finale en matière sèche de 15%. La protéine a été précipitée dans cette solution visqueuse en abaissant la valeur du pH à environ 2,5-3 à l'aide d'une solution aqueuse saturée d'acide trichloroacétique.

La suspension a été filtrée sous vide et le précipité a été mis en suspension deux fois dans 4 volumes d'eau distillée. Après centrifugation, les surnageants récupérés et réunis ont été concentrés par évaporation sous vide, dialysés et lyophilisés. D'une façon alternative, la solution concentrée a été directement lyophilisée, la poudre sèche lavée avec de l'éthanol pour éliminer de façon quantitative l'acide trichloroacétique résiduel et séchée une nouvelle fois sur un évaporateur rotatif.

Analyse:

L'activité d'inhibiteur de trypsine des extraits mesurée aux différentes étapes durant la purification, a été réalisée comme suit :

Solution A (tampon tris) : 6,05 g de tris(hydroxyméthyl)-aminométhane (Merck) et 2,94 g $CaCl_2 : 2H_2O$ dissous dans 900 ml d'eau. Le pH a été ajusté à 8,2 avec HCl et le volume ramené à 1 litre avec de l'eau.

Solution B : (trypsine): 10 mg de trypsine de porc, (NOVO Industrie, Copenhague) pesé exactement dissous dans 10 ml de HCl, 1mM.

Solution C (substrat) : 400 mg d'hydrochlorure de benzoyl-L-arginine-p-nitroanilide (L-BAPA) dissous dans 1 ml de sulfoxyde de diméthyle et dilués à 100 ml avec un tampon tris (solution A).

La réaction a été démarrée en ajoutant 500 ul de solution B à 2 ml de solution C et on a enregistré les changements d'absorbance résultants à 410 nm. Des quantités croissantes d'inhibiteur ont été ajoutées et les vitesses relatives après inhibition partielle ont été mesurées. Les points entre 0 et 50% d'inhibition ont été extrapolés de façon linéaire à la vitesse zéro et le volume correspondant de solution d'inhibiteur a été pris comme contenant une quantité stoechiométrique d'inhibiteur. Etant donné que les activités d'inhibiteur de l'inhibiteur de Bowman-Birk et de l'inhibiteur de Kunitz prédominant ne peuvent être distinguées, et que la quantité relative des inhibiteurs présents dans le soja est encore soumise à controverse, il n'est pas significatif d'exprimer l'activité d'inhibiteur trouvé dans les extraits en unité de poids d'inhibiteur.

Tableau 2.

| | Volume ml | Matière sèche g | Matière sèche % | Unités d'inhibiteur * |
|---|---|---|---|---|
| Extraits combinés de 500 g de farine de soja dégraissé avec 40% d'éthanol | 8200 | 48,8 | 9,76 | 22500 |
| Concentrat | 700 | 48,8 | 9,76 | 21900 |
| Rétentat UF | 75 | 6,8 | 1,36 | 19100 |
| coprécipité extrait | 100 | 0,6 | 0,12 | 1510 |
| lyophilisat | | 0,505 | 0,1 | 1510 |

\* 1 unité d'inhibiteur est définie comme la quantité d'activité inhibitrice susceptible d'inhiber 1 mg de trypsine.

La préparation finale d'inhibiteur de Bowman-Birk inhibe de façon stoechiométrique la trypsine commerciale avec l'activité la plus élevée, i.e. 1 mg d'inhibiteur inhibe 3 mg de trypsine.

L'analyse des acides aminés, réalisées sur un analyseur Chromacon (Kontron) conformément à des procédures standards, a révélé le nombre de 14 demi-cystines (20% du nombre total d'acides aminés) caractéristique des inhibiteurs du type Bowman-Birk.

8

Analyse de l'azote (Carlo Erba) : valeur 15,2% (théoriquement 16%).

La préparation d'inhibiteur de Bowman-Birk a été caractérisée, en terme de pureté et de taille moléculaire par électrophorèse sur gel de polyacrylamide en présence de 0,1% de dodécyl sulfate de sodium sur un gel de 12,5% (20 x 20 cm) avec un gel d'entassement de 3%. Préalablement à l'électrophorèse, les échantillons (0,1 mg) ont été réduits avec du mercaptoéthanol-2. Les poids moléculaires standards incluaient lipoxydase (PM 98 000), béta-amylase (PM 61 000), lectine de soja (PM 30 000), inhibiteur de trypsine de soja de Kunitz (PM 21 000) et alpha lactalbumine (PM 14 800). Le gel a été colorié avec du bleu de Coomassie G-250 dans de l'acide acétique à 7%.

Selon ce critère, l'inhibiteur de Bowman-Birk était pur, constitué, en accord avec la littérature, de diverses sous-espèces homologues.

Exemple 2.

Isolation d'inhibiteur de trypsine/chymotrypsine de poumon de bovin.

2,5 kg de poumon de bovin congelé ont été coupés en morceaux et homogénéisés dans un mélangeur de Waring. Le mélange homogène résultant a été mis en suspension durant une nuit dans 5 litres d'une solution contenant 8 parties d'éthanol et 2 parties d'eau (vol/vol). La suspension a ensuite été diluée avec 2,5 l d'eau et ensuite centrifugée pendant une heure. Le sédiment a été extrait avec 5 litres d'un mélange éthanol/eau à 50% et les surnageants ont été réunis et concentrés dans un évaporateur rotatif, la température n'excèdant pas 50°C.

Subséquemment, on a ajouté 15 g de caséinate de sodium à 250 ml d'extrait concentré, et le pH a été ajusté à 3,5 par addition rapide l'acide trichloroacétique.

Le précipité a été récupéré par filtration sous vide, extrait deux fois avec 100 ml d'eau distillée, et les surnageants concentrés.

L'extrait final contenant l'inhibiteur a été caractérisé de la même façon qu'à l'exemple 1, et selon ces critères s'est avéré homogène avec une activité maximale.

Exemple 3.

Purification d'alpha lactalbumine de colostrum de vache.

200 ml de colostrum de bovin ont été dégraissés par centrifugation et on a éliminé la caséine par centrifugation à vitesse élevée.

Le lait a été concentré cinq fois par ultrafiltration (membrane UM 5 de Diaflo) et diafiltré avec deux volumes supplémentaires (80 ml) d'eau.

Subséquemment, on a ajouté 4g de caséinate de sodium et après agitation pendant 1 heure, le pH a été amené à une valeur de 3,5 par addition rapide d'acide trichloroacétique.

Le précipité formé a été filtré au moyen d'une pompe à vide et extrait deux fois avec de l'eau distillée.

L'extrait a été travaillé et caractérisé comme décrit dans l'exemple 1.

L'analyse par électrophorèse a révélé la seule présence d'alpha lactalbumine. L'inhibiteur de trypsine colostral n'était pas détectable, bien que sa présence pouvait être révélée dans l'essai d'inhibition de trypsine.

**Revendications**

1. Procédé d'isolation d'un protide biologiquement actif, stable en milieu acide, d'origine animale, végétale ou microbienne, contenant moins d'environ 10% en poids d'agents contaminants non protéiniques, caractérisé en ce qu'il comprend :

la co-précipitation par acidification, en solution ou en suspension, dudit protide à isoler et d'au moins une protéine sensible à l'acide, présentant un poids moléculaire supérieur à celui dudit protide à isoler et supérieur à 20 000 et un point isoélectrique compris entre 1 et 5, et étant insoluble à son point isoélectrique ;

l'isolation dudit protide du précipité ainsi formé, par mise en suspension, avec une solution aqueuse, dudit précipité et séparation subséquente des matières insolubles en suspension ;

et éventuellement l'élimination subséquente des contaminants de faible poids moléculaire.

**2.** Procédé selon la revendication 1, caractérisé en ce qu'il consiste, préalablement à l'étape de co-précipitation précitée, à purifier le protide stable à l'acide, jusqu'à un degré de pureté d'au moins 1%, et de préférence supérieur à 10%, par toute opération de purification habituelle, à l'exception des opérations basées sur des techniques chromatographiques, telles que perméation sur gel, échange d'ions, chromatographie par affinité.

**3.** Procédé selon la revendication 1 ou 2, caractérisé en ce que la protéine sensible à l'acide précitée, est soit exogène, comme par exemple une protéine du lait ajoutée à un extrait de pancréas de porc, soit endogène, comme par exemple une protéine de soja ajoutée à un extrait de soja.

**4.** Procédé selon l'une des revendications 1 à 3, caractérisé en ce que la protéine sensible à l'acide précitée est ajoutée à une solution ou suspension contenant le protide à isoler sois forme sèche ou sous forme d'une solution concentrée, en une quantité telle que sa concentration finale en poids soit de 1 à 15%, et de préférence de 5 à 10%.

**5.** Procédé selon l'une des revendications 1 à 4, caractérisé en ce que l'opération d'acidification précitée est réalisée par addition à la solution de suspension contenant le protide à séparer et au moins une protéine sensible à l'acide, d'un acide jusqu'à l'obtention d'un pH compris entre 0 et 5, et de préférence compris entre 1,5 et 3,5.

**6.** Procédé selon la revendication 5, caractérisé en ce que l'acide précité est de préférence un acide susceptible de dénaturer la protéine sensible à l'acide précitée, et en particulier l'acide trichloroacétique.

**7.** Procédé selon la revendication 1, caractérisé en ce qu'il consiste, lors de l'étape d'isolation du protide précitée, à séparer les matières insolubles en suspension par centrifugation ou filtration et à concentrer la phase soluble contenant ledit protide par évaporation, en obtenant ainsi un concentrat ne contenant pratiquement pas de contaminants à poids moléculaire élevé et moins d'environ 10% en poids de matière dialysable à faible poids moléculaire.

**8.** Procédé selon l'une des revendications 1 à 7, caractérisé en ce que le protide stable à l'acide présente, préalablement à l'étape de co-précipitation précitée, une concentration d'au moins 1%, et de préférence de plus de 10%, basée sur la matière sèche.

**9.** Procédé selon l'une des revendications précédentes, caractérisé en ce que le protide précité à isoler est un inhibiteur de protéase polyvalent à faible poids moléculaire, tel que en particulier l'inhibiteur de Bowman-Birk et ses homologues génétiques.

**Claims**

**1.** Method of isolation of a biologically active protid stable in an acid medium, from animal, vegetable or microbial origin, containing less than about 10% by weight of non-proteinic contaminant agents, characterized in that it comprises :
the co-precipitation, by acidification in a solution or in suspension, of the said protid to be isolated and of at least one protein sensitive to the acid, exhibiting a molecular weight greater than that of the said protid to be isolated and greater than 20,000 and an isoelectric point comprised between 1 and 5 and being insoluble at its isoelectric point ;
the isolation of the said protid from the precipitate thus formed by suspension of the said precipitate with an aqueous solution and subsequent separation of the insoluble materials being in suspension ;
and possibly the subsequent elimination of the contaminants of low molecular weights.

**2.** Method according to claim 1, characterized in that it consists, prior to the aforesaid co-precipitation step, in purifying the acid-stable protid up to a degree of purity of at least 1% and preferably higher than 10% by any usual purification operation, with the exception of the operations based upon chromatographic techniques such as the permeation on a gel, ion exchange, chromatography through affinity.

**3.** Method according to claim 1 or 2, characterized in that the aforesaid protein sensitive to the acid is either exogenous for example like a milk protein added to an extract of pig pancreas or endogenous for example like a soy protein added to a soy extract.

**4.** Method according to one of claims 1 to 3, characterized in that the aforesaid protein sensitive to the acid is added to a solution or suspension containing the protid to be isolated in a dry form or in the form of a concentrated solution in such an amount that its final concentration by weight be from 1 to 15% and preferably from 5 to 10%.

**5.** Method according to one of claims 1 to 4, characterized in that the aforesaid acidification step is carried out by the addition to the suspension solution containing the protid to be separated and at least one protein sensitive to the acid, of an acid until the obtaining of a pH comprised between 0 and 5 and preferably comprised between 1.5 and 3.5.

**6.** Method according to claim 5, characterized in that the aforesaid acid preferably is an acid capable of denaturing the aforesaid protein sensitive to the acid and in particular the trichloroacetic acid.

**7.** Method according to claim 1, characterized in that it consists during the step of isolation of the aforesaid protid in separating the insoluble materials being in suspension by centrifugation or filtration and in concentrating the soluble phase containing the said protid by evaporation, thus obtaining a concentrate practically containing no contaminants with a high molecular weight and less than about 10% by weight of dialysable material with a low molecular weight.

**8.** Method according to one of claims 1 to 7, characterized in that the acid-stable protid exhibits prior to the aforesaid step of co-precipitation, a concentration of at least 1% and preferably of more than 10%, based upon the dry material.

**9.** Method according to one of the foregoing claims, characterized in that the aforesaid protid to be isolated is a polyvalent protease inhibitor with a low molecular weight such in particular as the Bowman-Birk inhibitor and its genetic homologues.

**Patentansprüche**

**1.** Verfahren zum Isolieren eines in einem sauren Medium stabilen, biologisch aktiven, weniger als ungefähr 10 % an kontaminierenden, nicht proteinischen Wirkstoffen enthaltenden Protids tierischer, pflanzlicher oder mikrobiellen Herkunft, dadurch gekennzeichnet, dass es umfasst :
das Mitniederschlagen, durch Säuerung, in einer Lösung oder in Suspension, des besagten zu isolierenden Protids und wenigstens eines gegenüber der Säure empfindlichen Proteins, das ein Molekulargewicht grösser als dasjenige des besagten zu isolierenden Protids un grösser als 20 000 und einen zwischen 1 und 5 liegenden isoelektrischen Punkt aufweist und bei seinem isoelektrischen Punkt unlöslich ist ;
das Isolieren des besagten Protids von dem somit gebildeten Niederschlag durch Aufschlämmung des besagten Niederschlags mit einer wässerigen Lösung und nachfolgenden Trennung der unlöslichen aufgeschlämmten Stoffen ;
und ggf. die nachfolgende Beseitigung der kontaminierenden Wirkstoffe schwachen Molekulargewichtes.

**2.** Verfahren nach Anspruch 1, dadurch gekennzeichnet, dass es darin besteht, vor dem besagten Vorgang des Mitfällens, den gegenüber Säure beständigen Protid bis zum einem Reinigungsgrad von wenigstens 1% und vorzugsweise grösser als 10% durch jeden üblichen Reinigungsvorgang mit der Ausnahme der sich auf chromatographische fachtechnische Verfahren stützenden Vorgänge, wie Permeation auf einem Gel, Ionenaustausch, Chromatographie durch Affinität zu reinigen.

**3.** Verfahren nach Anspruch 1 oder 2, dadurch gekennzeichnet, dass das vorgenannte gegenüber der Säure empfindliche Protein entweder exogen wie zum Beispiel ein einem Extrakt aus der Bauchspeicheldrüse des Schweines zugesetztes Protein der Milch oder endogen wie zum Beispiel ein einen Sojabohnenextrakt zugesetztes Sojabohnenprotein ist.

4. Verfahren nach einem der Ansprüche 1 bis 3, dadurch gekennzeichnet, dass das gegenüber der Säure empfindliche vorgenannte Protein einer der in trockner Form oder in der Form einer konzentrierten Lösung zu isolierenden Protid enthaltenden Lösung oder Aufschlämmung in einer derartigen Menge zugesetzt wird, dass seine Endgewichtskonzentration entweder 1 bis 15% und vorzugsweise 5 bis 10% beträgt.

5. Verfahren nach einem der Ansprüche 1 bis 4, dadurch gekennzeichnet, dass der vorgenannte Säuerungsvorgang durch Zusatz einer Säure der den abzuscheidenden Protid und wenigstens ein gegenüber der Säure empfindliches Protein enthaltenden Aufschlämmungslösung bis zur Erzielung eines zwischen 0 und 5 und vorzugsweise zwischen 1,5 und 3,5 liegendes pH durchgeführt wird.

6. Verfahren nach Anspruch 5, dadurch gekennzeichnet, dass die vorgenannte Säure vorzugsweise eine Säure und insbesondere die Trichloroessigsäure, die fähig ist, das gegenüber der vorgannten Säure empfindliche Protein zu verfälschen, ist.

7. Verfahren nach Anspruch 1, dadurch gekennzeichnet, dass es darin besteht, während dem Vorgang der Isolierung des vorgenannten Protids, die aufgeschlämmten unlöslichen Stoffe durch Zentrifugieren oder Filtrieren abzuscheiden und die den besagten Protid enthaltende lösliche Phase durch Verdunstung zu konzentrieren, wobei somit ein praktisch keine kontaminierende Wirkstoffe mit hohem Molekulargewicht und weniger als ungefähr 10 Gewichts-% eines dialysierbaren Stoffes mit schwachem Molekulargewicht enthaltendes Konzentrat gewonnen wird.

8. Verfahren nach einem der Ansprüche 1 bis 7, dadurch gekennzeichnet, dass der gegenüber der Säure beständige Protid vor dem vorgenannten Mitfällungsvorgang eine Konzentration auf Trockenstoffbasis von wenigstens 1% und vorzugsweise von mehr als 10% aufweist.

9. Verfahren nach einem der vorangehenden Ansprüche, dadurch gekennzeichnet, dass der zu isolierende vorgenannte Protid ein mehrwertiger Protease-Hemmstoff mit schwachem Molekulargewicht, wie insbesondere der Bowman-Birk'scher Hemmstoff und seine genetischen Homologuen ist.